Europäisches Patentamt

European Patent Office

Office européen des brevets

(19)

(11) Veröffentlichungsnummer : **0 181 536**
**B1**

# (12) EUROPÄISCHE PATENTSCHRIFT

(45) Veröffentlichungstag der Patentschrift :
03.01.90

(21) Anmeldenummer : 85113424.7

(22) Anmeldetag : 23.10.85

(51) Int. Cl.$^5$ : **C 07 D295/02**

(54) Verfahren zur Herstellung von 1-Alkyl- oder 1-Cycloalkylpiperazinen.

(30) Priorität : 03.11.84 DE 3440195

(43) Veröffentlichungstag der Anmeldung :
21.05.86 Patentblatt 86/21

(45) Bekanntmachung des Hinweises auf die Patenterteilung : 03.01.90 Patentblatt 90/01

(84) Benannte Vertragsstaaten :
BE DE FR GB IT NL

(56) Entgegenhaltungen :
GB-A- 1 030 310
US-A- 2 058 547
IND. ENG. CHEM. PROD. RES. DEV., Band 20, 1981,
Seiten 688-693, American Chemical Society; W.
TYLER GELGER et al.: "Useful products from piperazine. Methylpiperazines and an amorphous dibasic
acid/piperazine polymer"
JOURNAL OF ORGANIC CHEMISTRY, Band 21, 1956,
Seiten 86,87; L.T. PLANTE et al.: "Alkylations with
alcohols and a raney nickel catalyst"
Die Akte enthält technische Angaben, die nach dem
Eingang der Anmeldung eingereicht wurden und die
nicht in dieser Patentschrift enthalten sind.

(73) Patentinhaber : BASF Aktiengesellschaft
Carl-Bosch-Strasse 38
D-6700 Ludwigshafen (DE)

(72) Erfinder : Mueller, Herbert, Dr.
Carostrasse 53
D-6710 Frankenthal (DE)
Erfinder : Voges, Dieter, Dr.
Richard-Wagner-Strasse 28
D-6800 Mannheim 1 (DE)
Erfinder : Lengsfeld, Wolfgang, Dr.
Woogstrasse 46
D-6703 Limburgerhof (DE)

## Beschreibung

Die vorliegende Erfindung betrifft ein neues Verfahren zur Herstellung von 1-Alkyl- oder 1-Cycloalkylpiperazinen oder 1-Piperazinyl-3-tetrahydrofuranylmethan durch Umsetzung von Piperazin mit Alkanolen oder Cycloalkanolen oder 3-Hydroxymethyltetrahydrofuran in Gegenwart von Wasserstoff und Katalysatoren auf der Grundlage von Nickel, unter erhöhtem Druck und bei erhöhter Temperatur.

Nach dem Vorschlag der DE-B-2 205 597 sollen die N-Alkylderivate von Morpholin, Piperazin, Piperidin, Hexamethylenimin oder Pyrrolidin durch drucklose Alkylierung mit aliphatischen Hydroxylverbindungen oder deren Ethern in Gegenwart dehydratisierend wirkender Katalysatoren auf Basis Siliciumdioxid hergestellt werden. Im Falle des Piperazin tritt dabei jedoch keine vollständige Umsetzung auf.

In der EP-A-34 480 wird die Herstellung von N-Alkylaminen oder N,N-Di-alkylaminen mittels spezieller Katalaysatoren beschrieben, die auf Triphenylphosphinkomplexen der Metalle Indium, Rhodium, Ruthenium, Osmium, Platin, Palladium und Rhenium basieren. Dabei wird u.a. auch Piperazin als ein mögliches Ausgangsprodukt erwähnt.

Nach der Lehre der US-A-4 152 353 sollen Alkohole, Aldehyde oder Ketone in Gegenwart eines Nickel und Kupfer enthaltenden Katalysators mit Aminierungsreagenzien zu Aminen umgesetzt werden. Als Aminierungsreagenzien dienen vorzugsweise Ammoniak und primäre Amine. Die Verwendung u.a. von Piperazin als Aminierungsreagenz ist in der Beschreibung erwähnt.

In dem dort beschriebenen Verfahren muß aber ein Mindestmolverhältnis Aminierungsreagenz : Alkohol von 1 : 1 bis 5 : 1 eingehalten werden. Vorzugsweise soll sogar bei einem Molverhältnis von 100 : 1 gearbeitet werden.

Schließlich wird in J. Org. Chem. 21, 86 (1956) die Herstellung von N-alkylsubstituierten Piperazinen, ausgehend u.a. von Piperazin und niederen Alkanolen in Gegenwart eines Nickelkatalysators im Autoklaven beschrieben. Das Molverhältnis Piperazin : Alkanol betrug 1 : 25 bis 1 : 36. Die erhaltenen Ausbeuten an N-mono- bzw. N,N'-dialkylsubstituierten Piperazinen sind in diesem Falle aber sehr unbefriedigend.

Da die Aufarbeitung von 1-Alkylpiperazinen in Anwesenheit von nicht umgesetztem Piperazin außerordentlich erschwert wird, bestand die Aufgabe der vorliegenden Erfindung darin, ein Verfahren bereitzustellen, das einen praktisch vollständigen Umsatz des eingesetzten Piperazins erlaubt und gleichzeitig die Bildung von 1,4-Dialkylpiperazinen auf ein vertretbares Mindestmaß reduziert.

Dementsprechend wurde ein Verfahren zur Herstellung von 1-Alkyl- oder 1-Cycloalkylpiperazinen oder 1-Piperazinyl-3-tetrahydrofuranylmethan durch Umsetzung von Piperazin mit Alkanolen oder Cycloalkanolen oder 3-Hydroxymethyltetrahydrofuran in Gegenwart von Wasserstoff und Katalysatoren auf der Grundlage von Nickel, unter erhöhtem Druck und bein erhöhter Temperatur, gefunden, das dadurch gekennzeichnet ist, daß man die Umsetzung bei einer Temperatur von 130 bis 190 °C und einem Druck von 5 bis 100 bar in Gegenwart von 5 bis 40 Gew.-% Wasser und ggf. in Gegenwart von 0,01 bis 1 Gew.-% anorganischen Basen, bezogen auf die Reaktionsmischung durchführt und dabei ein Molverhältnis Piperazin : Alkanol oder Cycloalkanol oder 3-Hydroxymethyltetrahydrofuran von 1 : 1,5 bis 1 : 8 einhählt.

Dieses Ergebnis ist überraschend, denn es war nicht vorhersehbar, daß unter den genannten Bedingungen die für die destillative Aufarbeitung notwendige Forderung eines 97 bis 100 %igen Piperazinumsatzes erfüllt wird.

Es ist weiterhin auch unerwartet, daß trotz eines großen Überschusses an Alkanol bzw. Cycloalkanol, die gewünschten monoalkylierten Produkte, nämlich 1-Alkyl-bzw. 1-Cycloalkylpiperazine mit guter Selektivität (im allgemeinen 60 bis 90 %) gebildet werden.

Das erfindungsgemäße Verfahren läuft in Gegenwart von Katalysatoren auf der Grundlage von Nickel ab.

Die Katalysatoren können nach bekannten Verfahren hergestellt werden, z. B. durch Imprägnieren von Trägern wie Kieselgel, Aluminiumoxid, Bimsstein oder Bleicherde mit den entsprechenden wäßrigen Salzlösungen und sich anschließender Trocknung, Calcinierung und Reduktion. Sie können ebenso auch durch die Methode der Copräzipitation bereitet werden. Auch trägerfreie Katalysatoren, z. B. Raney-Nickel sind für das Verfahren geeignet. Die Katalysatoren werden dabei fest angeordnet oder als Suspension verwendet.

Vorzugsweise verwendet man einen Hydrier/ Dehydrierkatalysator auf der Grundlage von Nickel. Besonders bevorzugt ist dabei die Verwendung von Raney-Nickel in suspendierter Form. Bei Einsatz von Nickelkatalysatoren werden, sowohl was die Wirtschaftlichkeit des Verfahrens als auch dessen Selektivität betrifft die besten Ergebnisse erzielt.

Als Alkylierungsmittel werden Alkanole oder Cycloalkanole verwendet.

Vorzugsweise setzt man solche Alkanole ein, die 1 bis 10 und insbesondere 2 bis 6 Kohlenstofatome im Molekül aufweisen, beispielsweise Methanol, Ethanol, Propanol, Isopropanol, Butanol, Isobutanol, Hexanol, Octanol oder 2-Ethylhexanol.

Als Cycloalkanole werden bevorzugt solche Verbindungen verwendet, die 3 bis 12 Kohlenstofatome im Molekül aufweisen, z. B. Cyclopropanol, Cyclopentanol, Cyclohexanol, 2-Methylcyclohexanol, 4-Ethylcyclohexanol oder Cyclododecanol.

Das Molverhältnis von Piperazin : Alkanol bzw. Cylcoalkanol beträgt dabei 1 : 1,5 bis 1 : 8, vorzugsweise 1 : 2 bis 1 : 5. Die Durchführung des Verfahrens mit einem Molverhältnis das größer als 1 : 8 ist, bringt keine weiteren Vorteile. Man beobachtet dabei einen Anstieg an 1,4-Dialkyl- bzw. 1,4-Dicycloalkylpiperazinen.

Das erfindungsgemäße Verfahren wird bei einer Temperatur von 130 bis 190 °C, vorzugsweise 140 bis 180 °C durchgeführt. Es soll ein Druck von 5 bis 100 bar, vorzugsweise 20 bis 80 bar eingehalten werden. Dabei ist zwischen einem unteren Druckbereich von 5 bis 65 bar, vorzugsweise 20 bis 65 bar und einem oberen Druckbereich von 65 bis 100 bar, vorzugsweise 65 bis 80 bar zu unterscheiden.

Erfindungsgemäß die Umsetzung in Gegenwart von Wasserstoff durchgeführt, dabei wird der genannte Gesamtdruck im wesentlichen durch den Partialdruck des Wasserstoffs bestimmt. Dies bedeutet also, daß man die Alkylierung des Piperazins in Gegenwart von soviel Wasserstoff durchführt, daß sich der gennante Druckbereich einstellt.

Wird die Umsetzung im oberen Druckbereich vorgenommen, so wird hierbei eine geringere Temperatur benötigt als im unteren Druckbereich. In der Regel genügt dann eine Temperatur von 140 bis 170 °C.

Die Durchführung des erfindungsgemäßen Verfahrens oberhalb eines Drucks von 100 bar ist zwar möglich, jedoch werden beim Arbeiten in diesem Druckbereich keine weiteren Vorteile mehr erzielt.

Das neue Verfahren wird in Gegenwart von 2 bis 40 Gew. % Wasser, bezogen auf die Reaktionsmischung, durchgeführt. Ist der Wassergehalt geringer als 2 Gew. %, so vermindert sich die erreichbare Selektivität merkbar. Bei Erhöhung des Wassergehalts über 40 Gew. % erfolgt eine Verlangsamung der Umsetzung, ohne daß dabei eine höhere Selektivität erzielt würde.

In manchen Fällen soll die Alkylierung von Aminen mit Alkoholen in Abwesenheit von Wasser erfolgen, um so hohen Umsatz und hohe Reaktionsgeschwindigkeit zu erzielen. So wird beispielsweise bei der Herstellung von tertiären Aminen aus sekundären Aminen und Alkohol das bei der Umsetzung gebildete Wasser im Maße seiner Entstehung aus dem flüssigen Reaktionsgemisch entfernt (vgl. dazu DE-A-2 645 712).

Im Gegensatz zu dieser Regel verläuft das erfindungsgemäße Verfahren in Gegenwart von Wasser mit höherer Reaktionsgeschwindigkeit und besserer Selektivität ab. Vom ökonomischen Standpunkt her ist dies ein nicht zu unterschätzender Vorteil des erfindungsgemäßen Verfahrens. Piperazin ist eine feste Verbindung, die aus Gründen der Arbeitshygiene nicht einfach zu handhaben ist. Nachdem erfindungsgemäß aber die flüssigen wäßrige Piperazinlösungen, mit gleich gutem Erfolg wie das feste Piperazin eingesetzt werden können, gestaltet sich der Alkylierungsprozeß ökonomisch besonders günstig.

Eine bevorzugte Ausführungsform des erfindungsgemäßen Verfahrens besteht darin, die Umsetzung des Piperazins in Gegenwart von 0,01 bis 1 Gew. %, vorzugsweise 0,01 bis 0,1 Gew. % anorganischer Basen, jeweils bezogen auf die Reaktionsmischung, durchzuführen.

Als anorganische Basen werden die Verbindungen verwendet, die sich von den Alkali- oder Erdalkalimetallen ableiten. Man verwendet die Oxide, Hydroxide oder Carbonate. Beispielsweise sind geeignet Natriumhydroxid, Kaliumhydroxid, Natriumcarbonat, Natriumhydrogencarbonat, Calciumhydroxid oder Calciumoxid.

Es können natürlich mehrere der besagten Verbingungen gleichzeitig bei der Umsetzung anwesend sein. Bevorzugt werden Alkalicarbonate. Die Basen können in fester, pulvriger Form oder auch in Form von z. B. konzentrierten Lösungen zusammen mit den Hydrier-/Dehydrierkatalysatoren benutzt werden.

Wie oben bereits beschrieben, wird im erfindungsgemäßen Verfahren Alkohol im Überschuß eingesetzt. Bei Durchführung der Umsetzung in technischem Maßstab bedeutet dies, daß man zweckmäßig einen Teil des jeweiligen Alkanols bzw. Cycloalkanols im Kreise führt. Das bei der destillativen Abtrennung des jeweiligen Akohols vom Zielprodukt anfallende Azeotrop kann anschießend ohne aufwendige Trocknung direkt in den Prozeß zurückgeführt werden.

Das erfindungsgemäße Verfahren kann in kontinuierlicher oder diskontinuierlicher Arbeitsweise durchgeführt werden.

Die Ausgangsprodukte Piperazin, vorzugsweise in Form seiner wäßrigen Lösung, and Alkohol sowie gegebenenfalls die anorganische Base werden gemischt und dem jeweiligen Katalysatorsystem zugeführt und in Gegenwart von Wasserstoff zur Reaktion gebracht.

Die Umsetzung kann prinzipiell auch in Gegenwart eines mit Wasser mischbaren Lösungsmittels, das sich unter den Reaktionsbedingungen inert verhält, vorgenommen werden. Beisielsweise sind Tetrahydrofuran, Dioxan oder Ethylenglykoldimethylether zu nennen.

Die üblicherweise bei der Alkylierung von Aminen mit Alkoholen getroffene Maßnahme, das bei der Reaktion entstehende Wasser laufend abzutrennen, kann im vorliegenden Fall unterbleiben.

Es wurde weiterhin gefunden, daß die Selektivität der Umsetzung von Piperazin zu 1-Alkyl- bzw. 1-Cycloalkylpiperazinen teilweise auch von der Katalysatorkonzentration abhängig ist. Wird die kritische Mindestmenge des Katalysators unterschritten, so fällt unabhängig von der Reaktionsgeschwindigkeit die Selektivität deutlich ab. Ebenso wird bei zu hoher Katalysatorkonzentration ein Abfall der Selektivität festgestellt.

Die kritische Mindestkonzentration an Katalysator wird zwar durch dessen spezifische Aktivität bestimmt, so daß eine allgemeine Angabe schwer möglich ist. Es kann jedoch gesagt werden, daß beispielsweise bei der Verwendung von suspendiertem Raney-Nickel die kritische Katalysatorkonzentration bei ca. 0,2 bis 1 Gew. %, bezogen auf das Reaktionsgemisch, liegt. Handelsübliches Raney-Nickel setzt man zweckmäßig in einer Konzentration, die zwischen 2 bis 5 Gew. %, bezogen auf das Reaktionsgemisch, liegt, ein.

Nach erfolgter Umsetzung läßt sich der Katalysator beispielsweise durch Filtration, Zentrifugieren oder Sedimentieren abtrennen und für viele weitere Umsetzungen verwenden. Benützt man diese Arbeitsweise, so kann die Katalysatoreinsatzzahl mit Leichtigkeit auf Werte unterhalb von 0,1 % abgesenkt werden.

Bei kontinuierlicher Betriebsweise an einem fest angeordneten Katalysator muß in entsprechender Weise dafür gesorgt werden, daß eine bestimmte Katalysatorbelastung (kg Reaktanden pro 1 Katalysatorvolumen and Zeiteinheit) nicht überschritten wird, da sonst die Selektivität der Umsetzung vermindert wird. Die Katalysatorbelastung ist ebenfalls vom verwendeten Katalysator abhängig und muß von Fall zu Fall ermittelt werden. Beispielsweise zeigt ein handelsüblicher Nickel-Trägerkatalysator (Träger: Magnesiumsilicat), der ca. 50 Gew. % Nickel, bezogen auf den Katalysator enthält eine Belastbarkeitsgrenze, die bei etwa 0,5 kg Reaktanden pro 1 Katalysator in der Stunde liegt. Am einfachsten ermittelt man die notwendige Mindestverweilzeit (bzw. Höchstbelastung des Katalysators), indem man feststellt, wie weit man diese Größen ausgehend von einer gegebenen Größe ohne Verschlechterung des Verfahrensergebnisses (d. h. der Selektivität) veringern kann.

Bei Einhaltung der beschriebenen Maßnahmen gelingt es, mittels des erfindungsgemäßen Verfahrens das eingesetzte Piperazin praktisch vollständig umzusetzen.

Dieser Aspekt ist von besonderer Bedeutung bei der Synthese von N-Alkylpiperazinen, die einen niederen Alkylrest ($C_1$- bis $C_6$) aufweisen, denn bei der Aufarbeitung der dort entstehenden Reaktionsgemische ist es sehr schwer, nicht umgesetztes oder möglicherweise im Überschuß vorhandenes Piperazin abzutrennen. Bei der destillativen Aufarbeitung stört nämlich insbesondere das vorhandene Wasser, das bei der Umsetzung gebildet wird und gegebenenfalls in gewisser Menge auch bereits von Anfang an zugegen war. Es treten dann unübersichtliche zum Teil mehrfache Azeotrope zwischen Piperazin, Monoalkylpiperazin and Dialkylpiperazin auf.

Wenn dagegen eine vollständige Umsetzung des Piperazins eintritt, hat bei der destillativen Aufarbeitung lediglich eine Trennung des monoalkylierten Piperazins (Zielprodukt) vom dialkylierten Piperazin (Nebenprodukt) zu erfolgen wobei die jeweiligen Zielprodukte in hoher Reinheit erhalten werden.

Die genannten 1-Alkyl- bzw. 1-Cycloalkylpiperazine sind wertvolle Zwischenprodukte, beispielsweise für die Synthese von Wirkstoffen oder Farbstoffen.

Die nachfolgenden Beispiele sollen die Erfindung näher eräutern. Die genannten Teile sind Gewichtsteile. Sie verhalten sich zu Volumenteilen wie Kilogramm zu Liter.

Beispiel 1

Eine Mischung aus 1400 Teilen Piperazin, 950 Teilen Wasser und 3128 Teilen Ethanol wurden mit 250 Teilen wasserfeuchtem Raney-Nickel (30 Gew. % Wasser) versetzt und in einem Druckgefäß unter Rühren bei einem Wasserstoffdruck von 60 bar und einer Temperatur von 175 °C zur Reaktion gebracht. Die Reaktionsdauer betrug 10 Stunden. Es wurde keine Wasserstoffaufnahme beobachtet. Nach dem Abkühlen wurde das Druckgefäß entspannt. Dann dekantierte man das Reaktionsgemisch ab, befreite es von Katalysatorresten und analysierte es. Die Destillation bei 1 mbar/180 °C zeigte, daß weniger als 0,5 Gew. %, bezogen auf das wasserfreie Reaktionsprodukt an nichtflüchtigen Nenbenprodukten entstanden sind. Das Reaktionsprodukt bestand (ohne Berücksichtigung von Wasser und Ethanol) aus 0,8 Gew. % nichtumgesetztem Piperazin, 55 Gew. % Ethylpiperazin, 43 Gew. % 1,4-Diethylpiperazin und 1,5 Gew. % nicht identifizierter Substanzen. Durch fraktionierte Destillation erhielt man das 1-Ethylpiperazin in reiner Form mit einem Gehalt von über 99,5 Gew. %.

Beispiel 2

Es wurde analog Beispiel 1 verfahren, jedoch dem Reaktionsgemisch zusammen mit dem Raney-Nickel noch 1,5 Teile Natriumcarbonat zugesetzt. In diesem Fall war die Umsetzung bereits nach 5 Stunden abgeschlossen. Der Gehalt an 1-Ethylpiperazin im Austrag (ohne Berücksichtigung von Wasser und Ethanol) betrug 75 Gew. %. Der Anfall an höher siedenden Produkten sank in die Gegend der Nachweisbarkeitsgrenze. Das bei dieser, wie auch der vorstehenden Umsetzung erhaltene zurückdestillierte wasserhaltige Alkoholazeotrop konnte ohne weitere Reinigung für Weitere Umsetzungen eingesetzt werden. Auch der sedimentierte Katalysator konnte wieder verwendet werden und zeigte auch nach 20 maliger Verwendung keinen Abfall der Reaktivität, wenn bei jedem Ansatz 1 Gew. % der ursprünglich zugebenen Alkalisalzmenge erneut zugesetzt wurde.

Analoge Ergebnisse erhielt man bei der Verwendung von 2 Teilen Calciumhydroxid anstelle von Natriumcarbonat.

Beispiel 3

Man arbeitete analog Beispiel 1, jedoch in Gegenwart von 0,5 Gew. % Calciumhydroxid, bezogen auf die Reaktionsmischung, und setzte anstelle von Ethanol mit 5032 Teilen Butanol um. Nach 5 Stunden erhielt man 1-Butylpiperazin mit einer Ausbeute von 70 Gew. % und 1,4-Dibutylpiperazin von 29,5 Gew. % im von Wasser und Butanol befreiten Reaktionsaustrag.

Beispiel 4

Man arbeitete analog Beispiel 1, jedoch in Gegenwart von einem Teil Kaliumcarbonat und setzte anstelle von Ethanol mit der äquivalenten Menge 3-Hydroxymethyltetrahydrofuran um.

Nach 10 Stunden enthielt man 74 Gew. % 1-Piperazinyl-3-tetrahydrofuranylmethan und 25 Gew. % der disubstituierten Piperazinverbindung.

## Patentansprüche

1. Verfahren zur Herstellung von 1-Alkyl- oder 1-Cycloalkylpiperazinen oder 1-Piperazinyl-3-tetrahydrofuranylmethan durch Umsetzung von Piperazin mit Alkanolen oder Cycloalkanolen oder 3-Hydroxymethyltetrahydrofuran in Gegenwart von Wasserstoff und Katalysatoren auf der Grundlage von Nickel, unter erhöhtem Druck und bei erhöhter Temperatur, dadurch gekennzeichnet, daß man die Umsetzung bei einer Temperatur von 130 bis 190 °C und einem Druck von 5 bis 100 bar in Gegenwart von 5 bis 40 Gew.-% Wasser und ggf. in Gegenwart von 0,01 bis 1 Gew.-% anorganischen Basen, bezogen auf die Reaktionsmischung durchführt und dabei ein Molverhältnis Piperazin : Alkanol oder Cycloalkanol oder 3-Hydroxymethyltetrahydrofuran von 1 : 1,5 bis 1 : 8 einhält.

2. Verfahren gemäß Anspruch 1, dadurch gekennzeichnet, daß man Alkanole verwendet, die 1 bis 10 Kohlenstoffatome im Molekül aufweisen.

3. Verfahren gemäß Anspruch 1, dadurch gekennzeichnet, daß man Cycloalkanole verwendet, die 3 bis 12 Kohlenstoffatome im Molekül aufweisen.

## Claims

1. A process for the preparation of a 1-alkyl- or 1-cycloalkylpiperazine or 1-piperazinyl-3-tetrahydrofuranylmethane by reacting piperazine with an alkanol or cycloalkanol or 3-hydroxymethyltetrahydrofuran in the presence of hydrogen and a catalyst based on nickel, at superatmospheric pressure and elevated temperature, wherein the reaction is carried out at from 130 to 190 °C and under from 5 to 100 bar in the presence of from 5 to 40 % by weight of water and in the presence or absence of from 0.01 to 1 % by weight of an inorganic base, based on the reaction mixture, and the molar ratio of piperazine to alkanol or cycloalkanol or 3-hydroxymethyltetrahydrofuran is kept at from 1 : 1.5 to 1 : 8.

2. A process as claimed in claim 1, wherein an alkanol of 1 to 10 carbon atoms is used.

3. A process as claimed in claim 1, wherein a cycloalkanol of 3 to 12 carbon atoms is used.

## Revendications

1. Procédé de préparation de 1-alkyl- ou 1-cycloalkylpipérazines ou de 1-pipérazinyl-3-tétrahydrofurannylméthane par réaction de la pipérazine avec des alcanols, des cycloalcanols ou le 3-hydroxyméthyl-tétrahydrofuranne en présence d'hydrogène et de catalyseurs à base de nickel, sous pression élevée et à température élevée, caractérisé en ce qu'on effectue la réaction à une température de 130 à 190 °C et sous une pression de 5 à 100 bar en présence de 5 à 40 % en poids d'eau et éventuellement en présence de 0,01 à 1 % en poids d'une base minérale, par rapport au mélange réactionnel, en maintenant un rapport molaire pipérazine : alcanol ou cycloalcanol ou 3-hydroxyméthyltétrahydrofuranne de 1 : 1,5 à 1 : 8.

2. Procédé selon la revendication 1, caractérisée en ce qu'on utilise des alcanols qui présentent de 1 à 10 atomes de carbone dans la molécule.

3. Procédé selon la revendication 1, caractérisé en ce qu'on utilise des cycloalcanols qui présentent de 3 à 12 atomes de carbone dans la molécule.